# EUROPEAN PATENT SPECIFICATION

(11) **EP 3 498 274 B1**
(45) Date of publication and mention of the grant of the patent: **13.12.2023**
(21) Application number: 17838690.0
(22) Date of filing: 08.08.2017
(51) Int. Cl.: A61K 31/341, A61P 35/00, A61P 35/04

(54) **APPLICATION OF PHOSPHODIESTERASE 4 INHIBITOR ZL-N-91 IN PREPARATION OF MEDICAMENT FOR TREATING PROSTATE CANCER PROLIFERATION AND METASTASIS**
ANWENDUNG DES PHOSPHODIESTERASE-4-INHIBITORS ZL-N-91 ZUR HERSTELLUNG EINES ARZNEIMITTELS ZUR BEHANDLUNG DER PROLIFERATION UND METASTASIERUNG VON PROSTATAKREBS
APPLICATION DE ZL-N-91, INHIBITEUR DE PHOSPHODIESTÉRASE 4, DANS LA PRÉPARATION DE MÉDICAMENTS POUR LE TRAITEMENT DE LA PROLIFÉRATION ET LES MÉTASTASES DES CELLULES CANCÉREUSES DE LA PROSTATE

(30) Priority: 10.08.2016 CN 201610652824
(43) Date of publication of application: 19.06.2019
(73) Proprietor: Shenzhen Han-Hui Biomedical Technology, Ltd, Shenzhen, Guangdong 518118 (CN)
(72) Inventor: ZHAO, Allan zijian, Nanjing Jiangsu 211100 (CN); GONG, Sijia, Nanjing Jiangsu 211100 (CN); LIN, Yan, Nanjing Jiangsu 211100 (CN); LI, Fanghong, Nanjing Jiangsu 211100 (CN); LI, Xiaoxi, Nanjing Jiangsu 211100 (CN); ZHOU, Sujin, Nanjing Jiangsu 211100 (CN); ZHAO, Zhenggang, Nanjing Jiangsu 211100 (CN)
(74) Representative: Straus, Alexander
(86) International application number: PCT/CN2017/096399
(87) International publication number: WO 2018/028560

(56) References cited:
- CN-A- 101 495 454
- CN-A- 102 716 110
- POWERS, G.L. et al.: "Phosphodiesterase 4D Inhibitors Limit Prostate Cancer Growth Potential", Mol Cancer Res., vol. 13, no. 1, 15 January 2015 (2015-01-15), pages 149-160, XP055568013, ISSN: 1541-7786
- GONG, SIJIA et al.: "Effects of a Selective Phosphodiesterase 4 Inhibitor, ZL-n-91, on the Human Prostate Cancer PC-3 Cells and Xenografts in Nude Mice", Progress in Modern Biomedicine, vol. 16, no. 24, 30 August 2016 (2016-08-30), pages 4601-4604, XP009513645, ISSN: 1673-6273, DOI: 10.13241/j.cnki.pmb.2016.24.001

## Description

### FIELD OF THE INVENTION

The present invention relates to the application of a phosphodiesterase 4 (PDE4) inhibitor, in particular to application of PDE4 inhibitor ZL-n-91. The invention belongs to the field of tumor biology.

### BACKGROUND OF THE INVENTION

Prostate cancer is the most common malignant tumor in males in Europe and the United States. Prostate cancer ranks first in incidence and second only to lung cancer in mortality among males in the United States. Prostate cancer incidence has risen continuously in recent years in China, and the degree of histological malignancy of Chinese patients is higher than that of the United States patients. According to the survey of relative survival rate of patients with urologic cancer in Shanghai, 80.0%-90.0% of patients have developed to advanced prostate cancer at the time of diagnosis in china, with 5-year survival rate less than 30%. Due to the large population in China, the number of patients with prostate cancer increases dramatically, so it is necessary to improve prostate cancer prevention and treatment. Traditional treatments for prostate cancer include surgery, endocrine therapy, and radiotherapy and chemotherapy, but the therapeutic effect is not satisfactory. For recurrent prostate cancer, endocrine therapy with androgen deprivation is often adopted. The prostate cancer generally develops androgen-independence after 2-5 years of continuous endocrine therapy. The currently used therapy for patients with androgen-independent prostate cancer includes chemotherapy, radiotherapy, internal exposure to radionuclides and bisphosphonate treatment, etc., but the efficacy is not satisfactory. The treatment of prostate cancer is in a dilemma, and it is urgent to develop new treatments. Therefore, scientists and clinical experts are actively exploring safer and more effective treatments.

Phosphodiesterases (PDEs) have the function of hydrolyzing intracellular second messenger cAMP or cGMP, affecting the signaling pathways mediated by these second messengers and regulating the cell functions. PDEs include 11 subtypes, of which PDE4 specifically hydrolyzes cAMP. PDE4 is mainly distributed in various inflammatory cells, including mast cells, macrophage lymphocytes, epithelial cells, etc. It is involved in related physiological and pathological processes, including promoting monocyte and macrophage activation, neutrophil infiltration, proliferation of vascular smooth muscle, vasodilation and myocardial contraction, etc., having effects on central nervous system functions, cardiovascular functions, inflammation/immune system, and cell adhesion. Studies have shown that PDE4 inhibitors (PDE4i) have the functions of anti-inflammation, anti-allergy, and anti-platelet activation. Its main mechanisms include the follows: 1) inhibiting the release of various inflammatory mediators/cytokines, and inhibiting the expressions of IL-4 and IL-5 genes; 2) inhibiting the activation of leukocytes (such as respiratory bursts) and inhibiting leukocyte migration; 3) inhibiting the expression or up-regulation of cell adhesion molecules (CAM); 4) inducing to produce cytokines with inhibitory activity, such as IL-6; 5) inducing apoptosis; 6) stimulating the release of endogenous hormones and catecholamines.

Although PDE4 inhibitors that are underdevelopment or have been developed are mainly target on chronic obstructive pulmonary disease (COPD), asthma, inflammatory bowel disease, arthritis, etc., many studies have shown that PDE4 inhibitors have remarkable inhibitory effect on malignant tumors. Patricia Goldhoff heterotransplanted human brain astroglioma cell U87 into nude mice, and PDE4 inhibitors prolonged the survival time of tumor-bearing mice. In 2006, Motoshi Narita found that PDE4i could inhibit the growth of human melanoma cells. Petros X.E. Mouratidis found that the addition of PDE4 inhibitors CC-8075 and CC-8062 to pancreatic cancer cells could reduce cell proliferation and increase apoptosis. CN 101495454 A discloses PDE4 inhibitors, pharmaceutical compositions and methods of treatment using the inhibitors that can be used to treat a variety of disorders including tumor progression. CN 102716110 A discloses a phosphodiesterase 4 inhibitor capable of avoiding vomiting. The inhibitor is named 1-(4-difluoro methoxy-3-(tetrahydrofuran-3-oxyl) phenyl)-3-dimethyl-1-ketone. Powers et al. (Powers Ginny L, Mol Cancer Res 2015 Jan;13(1):149-60 Epub Aug 22, 2014) examined the effects of PDE4D inhibitors on pathways that are critical in prostate cancer and/or downstream of cyclic AMP. NVP-ABE171 and cilomilast decreased cell growth. Prostate cancer xenografts grown in nude mice that were treated with cilomilast or NVP-ABE171 had decreased wet weight and increased apoptosis compared with vehicle-treated controls. The authors suggest the pharmacologic inhibition of PDE4D using small-molecule inhibitors is an effective option for prostate cancer therapy.

The existing PDE4 inhibitors mainly include Rolipram, Cilomilast, Roflumilast, etc. As Rolipram and Cilomilast may induce dizziness, headache and gastrointestinal adverse reactions such as nausea and vomiting, their applications are limited. One of the possible causes of gastrointestinal side effects is the poor specificity of PDE4 inhibitors, which inhibits the whole PDE family. For example, Cilomitast inhibits PDE4 with Ki of 92 nM, only 500 to 1000 times of Ki for PDE1, 2, 3, and 5. Therefore, high dose of Cilomilast may interact with other PDE family members to cause side effects. In fact, it is common for most PDE4 inhibitors to cause side effect of vomiting at high doses. Although Roflumilast has been approved by US FDA for the treatment of COPD and it reduces lung inflammation, resists oxidative stress, effectively relieves fibrosis of the lungs and enhances mucosal clearance and rebuilds the airways, etc., it may produce some adverse reactions, mainly including diarrhea, weight loss, nausea, atrial fibrillation and aggravation of mental illness (such as insomnia, anxiety, depression), etc.

### SUMMARY OF THE INVENTION

It is an object of the present invention to provide a composition comprising PDE4 inhibitor ZL-n-91 for use in the treatment to inhibit prostate cancer metastasis.

In order to achieve the above object, the present invention adopts the following technical solutions.

A composition comprising PDE4 inhibitor ZL-n-91 for use in the treatment to inhibit proliferation of prostate cancer is also disclosed but not within the scope of protection of the present invention.

In the present invention, *in vitro* tumor cell and subcutaneous xenograft models are used to study the pathophysiological effects of ZL-n-91. Experiments have confirmed that the inhibitor can significantly inhibit the proliferation of human prostate cancer PC-3 cells and the growth of transplanted tumors, which lays a foundation for preparing drugs which inhibit proliferation of prostate cancer.

A composition comprising PDE4 inhibitor ZL-n-91 for use in the treatment to suppress metastasis of prostate cancer is also within the scope of protection of the present invention.

The present invention studies the biological effects of ZL-n-91 using human prostate cancer cells PC-3. The experimental results show that the inhibitor can significantly inhibit the migration of prostate cancer cells, which lays a foundation for preparing drugs which suppress metastasis of prostate cancer.

A composition comprising PDE4 inhibitor ZL-n-91 for use in the treatment of proliferation and metastasis of prostate cancer is also within the scope of protection of the present invention.

The routes of administration for the above compositions preferably are oral, injection or inhalation.

The structural formula of the PDE4 inhibitor ZL-n-91 of the present invention is shown in the figure below.

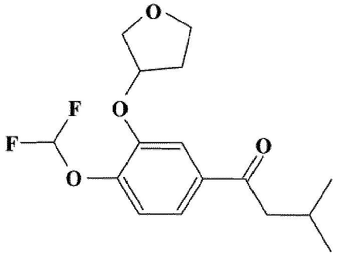

The PDE4 inhibitor ZL-n-91 of the present invention can be directly purchased or synthesized. For example, it can be synthesized with reference to the literature [Ruihong Ma, Bin-yan Yang, Chang-you Wu. A selective phosphodiesterase 4 (PDE4) inhibitor Zl-n-91 suppresses IL-17 production by human memory Th17 cells. International Immunopharmacology, 2008, 8(10): 1408-1417.]

In order to demonstrate the effects of the compounds in the present invention, in vivo xenograft growth inhibition experiments and in vitro cell experiments in mice are carried out in the following examples, to further elucidate the effects of PDE inhibitor ZL-n-91 of the present invention against proliferation and metastasis of prostate cancer.

The present invention can achieve the following beneficial effects. The selective PDE4 inhibitor ZL-n-91 of the present invention can significantly inhibit the proliferation and migration of tumor cells, indicating that the PDE4 inhibitor ZL-n-91 is expected to become an important target for inhibiting metastasis of prostate cancer. It will lay a foundation for preparing drugs against metastasis of prostate cancer, presenting good prospect of application. The inhibitory effect of ZL-n-91 on PDE4D is more than 5,000 times that of other PDE family members. Compared with other PDE4 inhibitors, this compound has higher selectivity, specificity and fewer side effects for PDE4D, which can effectively reduce or even avoid adverse reactions such as vomiting.

### BRIEF DESCRIPTION OF THE DRAWINGS

FIG. 1 shows the results of cell proliferation at 24 hours and 48 hours after treatment human prostate cancer cells PC-3 with different doses of ZL-n-91 respectively on the left and right figures.
FIG. 2 shows the change in body weights of nude mice subcutaneously implanted human prostate cancer cells PC-3 after treatment with ZL-n-91.
FIG.3 shows the change of tumor volume and tumor weight in nude mice with prostate cancer cell PC-3 subcutaneous xenograft after administration of ZL-n-91.
FIG.4 shows photograph of tumors from nude mice with prostate cancer cell PC-3 subcutaneous xenograft after administration of ZL-n-91.
FIG.5 shows the expression of Ki67 in tumor tissues. The left figure shows the immunohistochemistry results, the right figure shows the quantitative analysis.
FIG. 6 shows the migration of human prostate cancer cell PC-3 at 24 hours after administration of different doses of ZL-n-91.

### DETAILED DESCRIPTION OF THE EMBODIMENT

The present invention can be further understood in combination with the following examples. However, those skilled in the art should appreciate that the description of the embodiments is only intended to illustrate the invention and should not be construed as limiting the invention as described in the claims.

### Example 1: Effect of ZL-n-91 on proliferation of prostate cancer cells detected by CCK8 assay (additional information, not forming part of the invention)

1) PC-3 cells in logarithmic growth phase were taken to prepare single-cell suspension. 100ul cell suspension (containing 1×10⁴ cells) per well was plated into 96-well plates, and divided into 5 groups: the solvent control group, 10uM group, 50 uM group, 100 uM group, 200 uM group. Each group includes 6 replicates. Cells were pre-incubated for 24 hours (at 37° C, 5% CO₂);
2) Fresh medium was used, and ZL-n-91 at different concentrations was added to each group. cells were cultured continuously for 24 hours and 48 hours respectively (at 37 ° C, 5% CO₂ );
3) 100 ul of 10% CCK-8 solution was added to each well without air bubbles;
4) Cells were incubated for 1-2 hours, and the plates were taken out at 30 min, 60 min, and 90 min, respectively. The absorbance at 450 nm was measured using a microplate reader.

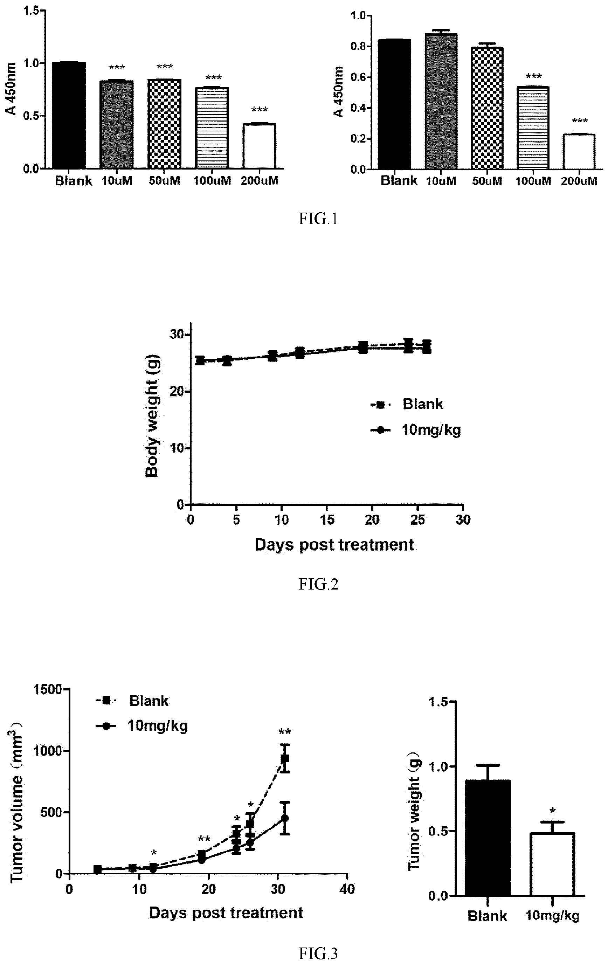

The results were shown in FIG. 1, indicating that the proliferation of human prostate cancer cells PC-3 was significantly decreased with the increase in the concentration of ZL-n-91.

### Example 2: Treatment of nude mice subcutaneously implanted with PC-3 cells

1) PC-3 cells in logarithmic growth phase were taken to prepare single-cell suspension in serum-free F-12K medium, and dispense 120ul aliquots per 1.5ml EP tubes (containing 2×10⁶ cells);
2) 0. 1ml of prepared cell suspension was subcutaneously inoculated into nude mice with a 1 ml sterile syringe;
3) Mice received drug treatment at 3 days after inoculation.
4) The nude mice were divided into two groups: the solvent control group and administration group (10 mg/kg). Mice received medication every day. The tumor volume and body weights of mice were measured twice a week;
5) When the tumor volume reached 1500 mm³, the tumor was taken out. The tumor size was measured, and the tumor weight was recorded.

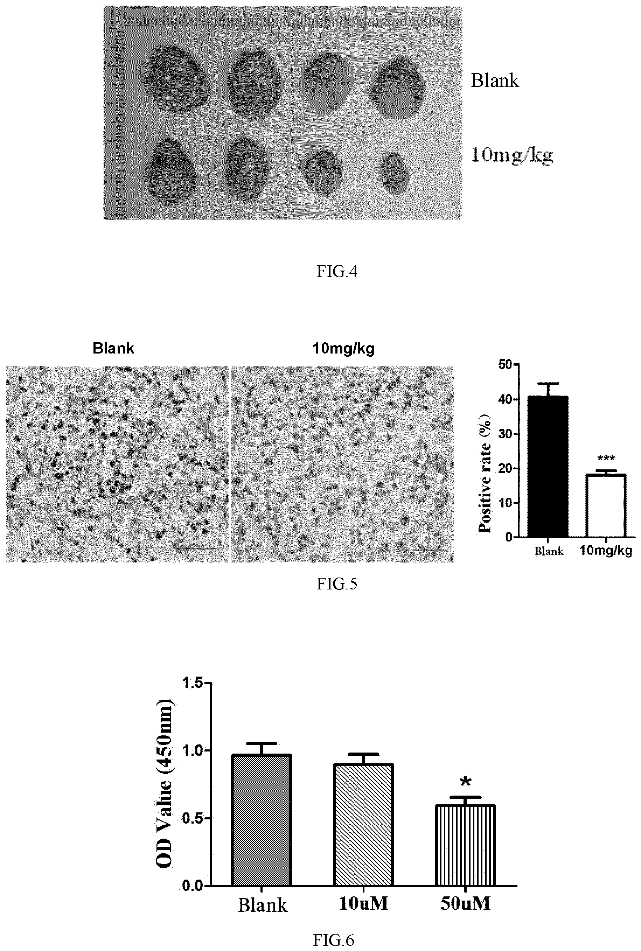

The results were shown in FIG.2. The results showed that the drug treatment had no effect on the body weight of mice. As shown in FIGS. 3 and 4, after the drug treatment, the tumor volume and tumor weight were about 1/2 of those in the control group, indicating that ZL-n-9 significantly inhibited the growth of PC-3 subcutaneous tumors.

### Example 3: Inhibition of the expression of Ki-67 in tumor tissues by ZL-n-91 (additional information, not forming part of the invention).

The resected tumors were fixed in 4% formalin overnight and embedded in paraffin for sectioning. Then the tumor proliferation antigen Ki67 was stained

As shown in FIG.5, the percentage of Ki67 positive cells in the tumor tissue of the drug administration group was significantly lower than that in the solvent control group, indicating that the proliferation of tumor cells in the drug administration group was decreased.

### Example 4: Effect of ZL-n-91 on the metastasis of PC-3 human prostate cancer cells

1) Cells in the logarithmic growth phase were taken to prepare suspension in serum-free DMEM medium. 100 ul of cell suspension per well (containing 5× 10⁴ cells) was added to the upper chamber of the Transwell. It was divided into 3 groups: the solvent control group, 10uM group, 50uM group. Each group includes three replicates.
2) The complete DMEM medium containing 10% FBS was added to the lower chamber of a 12-well plate.
3) 12 hours later, the upper chamber was taken out and fixed in ice methanol for 30 min, and dried naturally at room temperature.
4) 600 ul of 0.1% crystal violet solution was added to 12-well plate to stain the cells in the lower surface of the chamber for 15 min.
5) PBS was added to the chamber, and then pipetted out after 5 min, the PBS was. The cells from the top of the membrane were wiped gently with a clean cotton swab. The chambers were washed three times with PBS, then dried naturally at room temperature.
6) 300ul of 10% acetic acid solution was added to the 12-well plate, to soak the lower surface of the chamber for 10 min to dissolve the crystal violet particles in the cells.
7) 100 ul solution from each well of 12-well plate was placed to a 96-well plate, to measure the absorbance at 570 nm using a microplate reader.

The results were shown in FIG.6, which indicated that ZL-n-91 inhibited the metastasis of human prostate cancer PC-3 cells when the drug concentration was 50uM.

The above results indicate that the PDE4 inhibitor ZL-n-91 used in the present invention can inhibit the proliferation and metastasis of prostate cancer cells, presenting a good anti-tumor effect.

## Claims

1. Composition comprising a PDE4 inhibitor ZL-n-91 for use in a treatment to suppress metastasis of prostate cancer.

2. Composition comprising a PDE4 inhibitor ZL-n-91 for use according to claim 1, to target the proliferation and metastasis of prostate cancer.

3. Composition comprising a PDE4 inhibitor ZL-n-91 for use according to claims 1 to 2, wherein the composition can be administered via oral, injection or inhalation.

## Patentansprüche

1. Zusammensetzung, welche einen PDE4-Inhibitor ZL-n-91 umfasst, zur Verwendung bei einer Behandlung zur Unterdrückung der Metastasierung von Prostatakrebs.

2. Zusammensetzung, welche einen PDE4-Inhibitor ZL-n-91 umfasst, zur Verwendung nach Anspruch 1, zur gezielten Bekämpfung der Proliferation und Metastasierung von Prostatakrebs.

3. Zusammensetzung, welche einen PDE4-Inhibitor ZL-n-91 umfasst, zur Verwendung nach Ansprüchen 1 bis 2, wobei die Zusammensetzung oral, durch Injektion oder Inhalation verabreicht werden kann.

## Revendications

1. Composition comprenant un inhibiteur de la PDE4 ZL-n-91, à utiliser dans un traitement visant à supprimer les métastases du cancer de la prostate.

2. Composition comprenant un inhibiteur de la PDE4 ZL-n-91 à utiliser selon la revendication 1 pour cibler la prolifération et les métastases du cancer de la prostate.

3. Composition comprenant un inhibiteur de PDE4 ZL-n-91 à utiliser selon les revendications 1 à 2, dans laquelle la composition peut être administrée par voie orale, par injection ou par inhalation.
